# EUROPEAN PATENT APPLICATION

(11) **EP 1 920 772 A1**
(43) Date of publication of application: **14.05.2008**
(21) Application number: 06797031.9
(22) Date of filing: 30.08.2006
(51) Int. Cl.: A61K 31/41, A61K 31/50, A61P 29/00

(54) **PROPHYLACTIC AND/OR THERAPEUTIC METHOD FOR RHEUMATOID ARTHRITIS**

(30) Priority: 31.08.2005 US 712418 P
(71) Applicant: Kowa Company, Ltd., Naka-ku Nagoya-shi, Aichi-ken 460-8625 (JP)
(72) Inventor: TABUNOKI, Yuichiro, Tokyo 203-0051 (JP); KOSHI, Tomoyuki, Saitama 353-0006 (JP)
(74) Representative: Blodig, Wolfgang
(86) International application number: PCT/JP2006/317056
(87) International publication number: WO 2007/026737

(57) **Abstract**

The present invention provides a prophylactic and/or therapeutic agent for rheumatoid arthritis comprising 2-benzyl-5-(4-chlorophenyl)-6-[4-(methylthio)phenyl]-2H-pyr idazin-3-one and leflunomide in combination.

The prophylactic and/or therapeutic agent of the present invention can be administered orally, exhibits an excellent arthritis suppressive effect with fewer side effects, and is therefore useful for the prophylaxis and/or treatment of rheumatoid arthritis.

## Description

### Technical Field

The present invention relates to a prophylactic and/or therapeutic method for rheumatoid arthritis.

### Background Art

Rheumatoid arthritis is a disease in which inflammation accompanied by swelling or pain occurs in multiple joints and progresses over a long period of time, thereby irreversible deformation and functional disorder of joints leading to significant lowering of the quality of life (QOL). In Japan, there are patients corresponding in number to 0.6% of the total population, and 1% of the population of age over 30, and particularly in recent years, there is observed a tendency for an increase in the number of elderly rheumatoid patients, along with the progress of the aged society.

The disease stages of rheumatoid arthritis can be classified into four stages of the "initial phase, "a stage in which even though manifestation of joint pain or arthritis exists, definitive diagnosis of rheumatoid arthritis cannot be given yet; the "early phase," a stage in which definitive diagnosis of rheumatoid arthritis can be given, but there are no irreversible change, or, if any, the change is small(the early phase rheumatoid arthritis generally refers to 1 to 2 years from the onset of the disease); the "advanced phase" in which irreversible changes emerge, and systemic symptoms such as fatigue, mild fever and weight loss appear intensely; and the "late phase" in which inflammation in the joints is almost going to remission, but irreversible changes such as deformation and contracture still remain strong, with the predominant symptoms being pain and functional disorders. Thus, the therapeutic method varies with the respective stages of disease. With regard to the pathogenesis of rheumatoid arthritis, there have been reported research results that genetic factors or acquired factors (infection) are involved, but because the causes thereof have not been clarified, it is still impossible to achieve complete cure or prevention of the disease.

Therefore, the goal of therapy under the present circumstances lies in achieving early diagnosis of rheumatoid arthritis, suppressing the inflammation caused by rheumatoid arthritis rapidly and maximally as much as possible, and thus preventing the emergence of any irreversible changes or else inhibiting progress thereof, thereby attempting an improvement of the QOL of patients from physical, mental and social aspects. Therefore, during the therapy, patients are given sufficient explanation on the disease or the therapeutic methods for the disease, and then various means of treatment such as physical therapy, kinesitherapy, drug therapy and surgical therapy are applied to the patients.

In drug therapy, non-steroidal antiinflammatory drugs (NSAIDs), disease-modifying antirheumatic drugs (DMARDs) and steroid drugs are being used in the clinical field. Recently, biopharmaceuticals such as antibodies targeted against inflammatory cytokines are also used (Non-Patent Document 1).

DMARDs are classified as immunomodulators and immunosuppressants on the basis of their action mechanism, and among them, methotrexate (N-[4-[(2,4-diamino-6-pteridinyl)methylamino]benzoyl]-L-glu tamic acid), which is an immunosuppressant working on the mechanism of an antagonistic action against folic acid metabolism (dihydrofolate reductase inhibitory action), or leflunomide (N-(4-trifluoromethylphenyl)-5-methylisoxazole-4-carboxamid e) working on the mechanism of pyrimidine biosynthesis inhibitory action (dihydroorotate dehydrogenase (DHODH) inhibitory action), are acknowledged to have high anti-rheumatic effects. However, since both methotrexate and leflunomide can induce serious side effects including infection or interstitial pneumonia, these drugs have disadvantages that not only it is necessary to strictly control the method of administration or dose, but also sufficient investigation on the background of affection of the patient is required.

Furthermore, although biologics have potent antirheumatic effects, the biologics also have a disadvantage in the aspect of medical economics that they are expensive, as well as another disadvantage in the aspect of convenience that the route of administration thereof is limited to injection, and oral administration cannot be adopted.

Interleukin 1β (IL-1β), which is an inflammatory cytokine, is recognized with increased production thereof in a number of diseases such as, for example, rheumatoid arthritis, osteoarthritis, osteoporosis, inflammatory bowel disease, immune deficiency syndrome, septicemia, hepatitis, nephritis, ischemic diseases, insulin-dependent diabetes, arteriosclerosis, Parkinson's disease, Alzheimer's disease and leukemia, and is known to induce the synthesis of enzymes which are conceived to be associated with inflammation, such as collagenase, COX and PLA2. IL-1β is also known to cause articular destruction which is very similar to rheumatoid arthritis when intra-articularly injected to animals. Thus, IL-1β inhibitors are being studied and developed as drugs for treating inflammatory diseases, and there are known materials derived from biogenic substances such as IL-1 receptor antagonist (Non-Patent Document 2), and low molecular weight compounds such as T-614 (Non-Patent Document 3), S-2474 (Non-Patent Document 4), 2-benzyl-5-(4-chlorophenyl)-6-[4-(methylthio)phenyl]-2H-pyr idazin-3-one (Patent Document 1), and FR133605 (Non-Patent Document 5).

Pathological conditions of rheumatoid arthritis vary considerably among patients in terms of age, disease stage, complications, side effects, QOL and the like. Also, there exists no ultimate therapeutic drug, and even a phenomenon called "escape phenomenon" is observed, in which a therapeutic drug having a well established controlling effect on symptoms suddenly begins to lose the effect. Under such circumstances, when a drug therapy is to be performed, conversion of drugs or combined use of drugs is frequently employed, and thus clinical studies on the method of using such drugs are also being actively carried out. However, the effect of using the low molecular weight compounds exhibiting the IL-1β inhibitory effect, and pyrimidine biosynthesis inhibitors in combination has not been known heretofore.

[Patent Document 1] Pamphlet of International Patent Publication WO 99/25697
[Non-Patent Document 1] Arthritis & Rheumatism 46, pp 328-346, 2002
[Non-Patent Document 2] Arthritis & Rheumatism 42, pp 498-506, 1999
[Non-Patent Document 3] J. Pharmacobio-Dyn. 11, pp 649-655, 1992
[Non-Patent Document 4] YAKUGAKU ZASSHI 123, pp 323-330, 2003
[Non-Patent Document 5] J. Rheumatol. 23, pp 1778-1783, 1996

### Disclosure of the Invention

### Problems to be Solved by the Invention

It is an object of the present invention to provide a medicament having an excellent effect of treatment and/or prophylaxis of rheumatoid arthritis.

### Means for Solving the Problems

Under such circumstances as described above, the inventors of the present invention have devotedly conducted research, and as a result, found that an excellent arthritis suppressive effect can be obtained by using 2-benzyl-5-(4-chlorophenyl)-6-[4-(methylthio)phenyl]-2H-pyr idazin-3-one, which is an IL-1β inhibitor, and leflunomide, which is a pyrimidine biosynthesis inhibitor, in combination. Thus, the inventors completed the present invention.

That is, the present invention is to provide a prophylactic and/or therapeutic agent for rheumatoid arthritis, comprising 2-benzyl-5-(4-chlorophenyl)-6-[4-(methylthio)phenyl]-2H-pyr idazin-3-one and leflunomide in combination.

Furthermore, the present invention is to provide the use of 2-benzyl-5-(4-chlorophenyl)-6-[4-(methylthio)phenyl]-2H-pyr idazin-3-one and leflunomide for manufacturing a prophylactic and/or therapeutic agent for rheumatoid arthritis.

In addition, the present invention is to provide a prophylactic and/or therapeutic method for rheumatoid arthritis, comprising administering 2-benzyl-5-(4-chlorophenyl)-6-[4-(methylthio)phenyl]-2H-pyr idazin-3-one and leflunomide.

### Effect of the Invention

The prophylactic and/or therapeutic agent for rheumatoid arthritis of the present invention can be orally administered, exhibits an excellent inhibitory effect on arthritis with fewer side effects, and thus is useful in the prophylaxis and/or treatment of rheumatoid arthritis.

### Brief Description of the Drawings

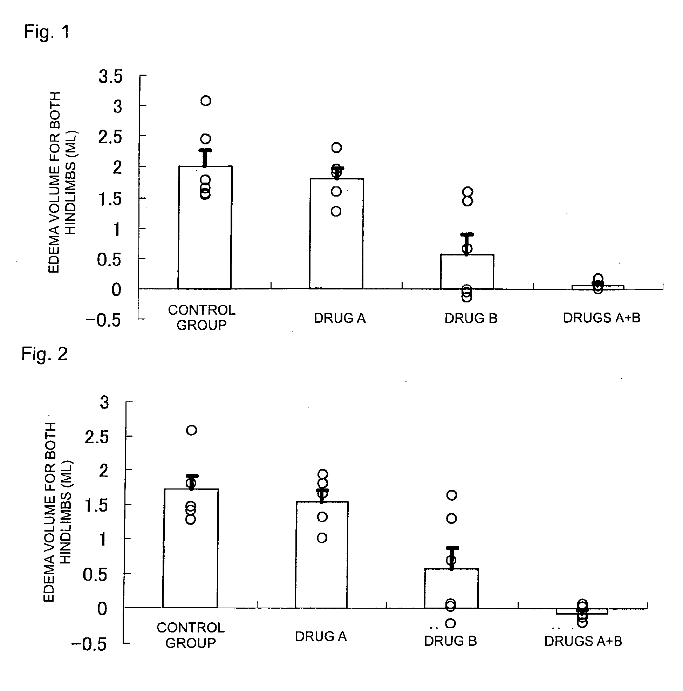

Fig. 1 is a diagram showing the edema volumes for both hindlimbs in a rat collagen-induced arthritis model after 18 days from the initial sensitization, for a control group (group of no drug administration), a group administered with 3 mg/kg of 2-benzyl-5-(4-chlorophenyl)-6-[4-(methylthio)phenyl]-2H-pyr idazin-3-one (Drug A), a group administered with 1.5 mg/kg of leflunomide (Drug B), and a group administered with Drugs A and B in combination.
Fig. 2 is a diagram showing the edema volumes for both hindlimbs in a rat collagen-induced arthritis model after 21 days from the initial sensitization, for a control group (group of no drug administration), a group administered with 3 mg/kg of 2-benzyl-5-(4-chlorophenyl)-6-[4-(methylthio)phenyl]-2H-pyr idazin-3-one (Drug A), a group administered with 1.5 mg/kg of leflunomide (Drug B), and a group administered with Drugs A and B in combination.

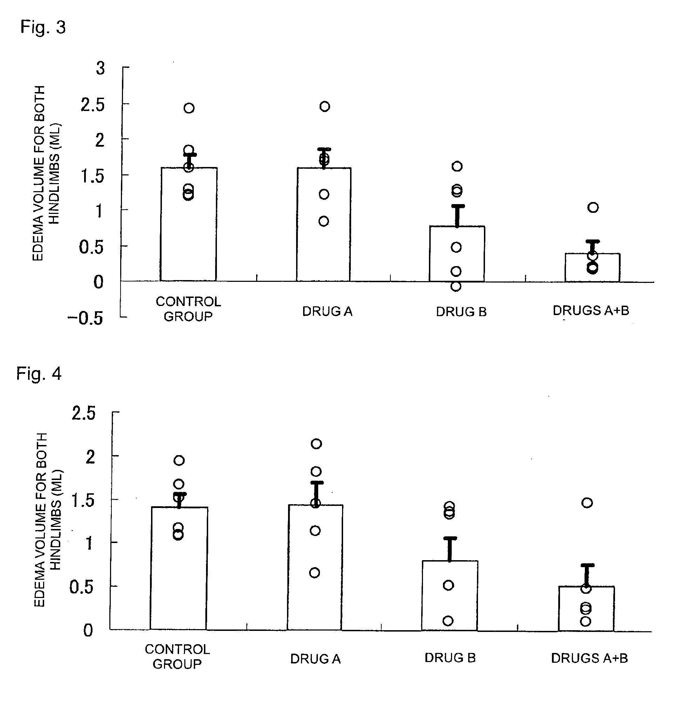

Fig. 3 is a diagram showing the edema volumes for both hindlimbs in a rat collagen-induced arthritis model after 26 days from the initial sensitization, for a control group (group of no drug administration), a group administered with 3 mg/kg of 2-benzyl-5-(4-chlorophenyl)-6-[4-(methylthio)phenyl]-2H-pyr idazin-3-one (Drug A), a group administered with 1.5 mg/kg of leflunomide (Drug B), and a group administered with Drugs A and B in combination.
Fig. 4 is a diagram showing the edema volumes for both hindlimbs in a rat collagen-induced arthritis model after 31 days from the initial sensitization, for a control group (group of no drug administration), a group administered with 3 mg/kg of 2-benzyl-5-(4-chlorophenyl)-6-[4-(methylthio)phenyl]-2H-pyr idazin-3-one (Drug A), a group administered with 1.5 mg/kg of leflunomide (Drug B), and a group administered with Drugs A and B in combination.

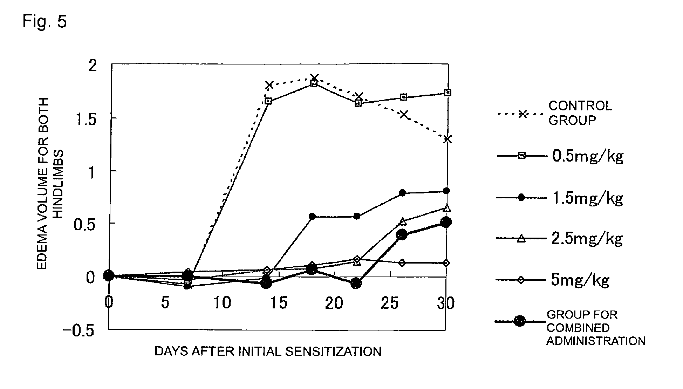

Fig. 5 is a diagram obtained by superposing the changes over time of the edema volumes for both hindlimbs of the dose response (0.5, 1.5, 2.5, 5 mg/kg) of leflunomide, with the changes over time of the same in the group administered with Drugs A and B in combination.

### Best Mode for Carrying Out the Invention

2-Benzyl-5-(4-chlorophenyl)-6-[4-(methylthio)phenyl]-2H-pyridazin-3-one (hereinafter, may also be described as Drug A), which is used as a prophylactic and/or therapeutic agent for rheumatoid arthritis of the present invention, is a known substance, and can be produced by, for example, the method described in International Patent Publication WO 99/25697, or a similar method. That is, p-chlorophenylacetic acid is reacted with thioanisole using a condensing agent such as polyphosphoric acid, thus to obtain 2-(4-chlorophenyl)-4'-(methylthio)acetophenone (compound a). Compound "a" and a base such as potassium t-butoxide are allowed to react in tetrahydrofuran, and then ethyl bromoacetate is added to obtain ethyl 2-(4-chlorophenyl)-4-[4-(methylthio)phenyl]-4-oxobutanoate (compound b). Compound b and hydrazine hydrate are allowed to react in ethanol to obtain 5-(4-chlorophenyl)-6-[4-(methylthio)phenyl]-4,5-dihydro-2H-pyridazin-3-one (compound c). Compound c and bromine are allowed to react in acetic acid, and the product is dehydrogenated, to obtain 5-(4-chlorophenyl)-6-[4-(methylthio)phenyl]-2H-pyridazin-3-one (compound d). Compound d thus obtained and benzyl bromide are allowed to react in a solvent such as N,N-dimethylformamide using a base such as potassium carbonate, and thus Drug A can be produced.

For leflunomide, which is used as a prophylactic and/or therapeutic agent for rheumatoid arthritis of the present invention, commercially available products, for example, products of KEMPROTEC, Ltd., can be used.

As described in the Examples below, it was found that combined administration of Drug A and leflunomide synergistically suppresses edema in both hindlimbs, thus suppressing arthritis. Therefore, a medicament comprising Drug A and leflunomide in combination is useful as a prophylactic and/or therapeutic agent for rheumatoid arthritis, and in particular, rheumatoid arthritis accompanied by articular inflammation.

The ratio of combination of Drug A and leflunomide in the prophylactic and/or therapeutic agent for rheumatoid arthritis of the present invention is preferably in the range of 100:1 to 1:10, and more preferably 60:1 to 1:1, by mass (Drug A: leflunomide), from the viewpoint of the arthritis suppressive effect.

The prophylactic and/or therapeutic agent for rheumatoid arthritis of the present invention can be provided as a kit comprising a medicament containing Drug A and a medicament containing leflunomide, or as a combination preparation containing Drug A and leflunomide. That is, pharmaceutical preparations respectively containing the active ingredients, the Drug A and leflunomide, may be prepared, and those preparations may be administered simultaneously or separately at a time interval, or administered as a combination preparation.
The dosage form of the medicament of the present invention is not particularly limited, and can be appropriately selected according to the purpose of therapy. For example, there may be mentioned oral administration using tablets, capsules, granules, film-coated tablets, powders, syrups or the like, or parenteral administration using injections, suppositories, inhalants, percutaneously absorbed preparations, eye drops, nasal drops or the like, but oral administration is preferred.

A pharmaceutical preparation suitable for these dosage forms may be appropriately combined with pharmaceutically acceptable carriers, for example, diluents or bulking agents such as starches, lactose, sucrose, mannitol, and silicic acid; disintegrants such as agar, calcium carbonate, potato or tapioca starch, alginic acid, and specific complex silicates; binder such as hydroxypropylmethylcellulose, alginates, gelatin, polyvinylpyrrolidone, sucrose, and gum arabic; lubricants such as talc, calcium stearate, magnesium stearate, solid polyethylene glycols, sodium lauryl sulfate, and mixtures thereof; diluents such as lactose and corn starch; buffers of organic acids such as citric acid, phosphoric acid, tartaric acid and lactic acid, of inorganic acids such as hydrochloric acid, of alkali hydroxides such as sodium hydroxide and potassium hydroxide, and of amines such as triethanolamine, diethanolamine and diisopropanolamine; antiseptics such as paraoxybenzoic acid esters and benzalkonium chloride; emulsifying agents, for example, anionic surfactants such as calcium stearate, magnesium stearate and sodium lauryl sulfate, cationic surfactants such as benzalkonium chloride, benzetonium chloride and cetylpyridinium chloride, and nonionic surfactants such as glyceryl monostearate, sucrose fatty acid esters, polyoxyethylene hardened castor oil, polyoxyethylene sorbitan fatty acid esters, polyoxyethylene fatty acid esters and polyoxyethylene alkyl ethers; and stabilizing agents such as sodium sulfite, sodium hydrogen sulfite, dibutylhydroxytoluene, butylhydroxyanisole, and EDTA. In addition, odor-suppressors, dispersants, preservatives, flavoring agents and the like may also be appropriately combined and used, as required.

The dosage of 2-benzyl-5-(4-chlorophenyl)-6-[4-(methylthio)phenyl]-2H-pyr idazin-3-one of the present invention is appropriately selected on the basis of the weight, age, gender, symptoms and the like of the patient, but typically, a daily dose for an adult may be 2 to 320 mg, and preferably 4 to 160 mg, per day. Also, the dosage of leflunomide is appropriately selected on the basis of the weight, age, gender, symptoms and the like of the patient, but typically, a daily dose for an adult may be 5 to 100 mg, and preferably 10 to 100 mg, per day. Furthermore, the administration may be carried out once a day, or in two or more divided portions a day.

### EXAMPLES

Hereinafter, the present invention will be described in more detail with reference to Examples, but the present invention is not intended to be limited to these Examples.

### EXAMPLE 1

For the cases of combined administration of 2-benzyl-5-(4-chlorophenyl)-6-[4-(methylthio)phenyl]-2H-pyr idazin-3-one (the product synthesized by the above-described method is used) (Drug A) and leflunomide (Drug B), and respective single drug administrations, the effect of edema suppression in both hindlimbs was determined by the following method (rat collagen-induced arthritis model) (FDA, CBER, CDER, CDRH: Guidance for industry - Clinical development programs for drugs, devices, and biological products for the treatment of rheumatoid arthritis (RA)-. (1999)). In addition, the test animal used was female Lewis rat (LEW/Crj) (obtained from Charles River Laboratories Japan, Inc.).

For each 8-week-old LEW/Crj rat, the volume of a portion extending from the ankle to the paw was measured for both hindlimbs (hereinafter, hindlimbs volume) by means of a plethysmometer for small animals (TK-101CMP, product of Unicom), and the total volume was taken as the hindlimbs volume upon initiation of test (hereinafter, Pre value). This Pre value was used as an index to divide the rats into groups by one-parameter-based block randomization, so that the values in the respective groups averaged out.
The collagen emulsion for sensitization that was used to induce arthritis in rats, was prepared by homogenizing a 0.3% Type II collagen solution (Collagen Research Center), Adjuvant Peptide (Peptide Institute, Inc.) and Adjuvant Incomplete Freund (DIFCO), using a Handy Micro Homogenizer (Microtec Nition, Co., Ltd.) under ice-cooling. The collagen emulsion thus prepared was intracutaneously administered at 10 sites in the back of each rat at a dose of 0.1 mL/site, to perform initial sensitization of the rat. Seven days after the initial sensitization, 0.12 mL of the same collagen emulsion was intracutaneously administered to the tail head of each rat, to perform booster sensitization.
Drug administration was conducted from the day after the initial sensitization through 29 days thereafter. The group for sole administration of Drug A was orally administered with the drug at 3 mg/kg, twice a day, in the morning (9:00 to 11:00) and in the evening (15:30 to 17:30). The group for sole administration of Drug B was orally administered with the drug at 1.5 mg/kg, once a day, in the afternoon (11:30 to 13:30). On the other hand, the group for combined administration of Drug A and Drug B was orally administered with Drug A at 3 mg/kg in the morning (9:00 to 11:00) and in the evening (15:30 to 17:30), and with Drug B at 1.5 mg/kg in the afternoon (11:30 to 13:30).
14, 18, 21, 26 and 31 days after the initial sensitization, the hindlimbs volume was measured again for each rat, and the difference between each of the values and the Pre value was determined to calculate the edema volume for both hindlimbs.

Tables 1 to 4 and Figs. 1 to 4 represent the edema volume for both hindlimbs after 18, 21, 26 and 31 days from the initial sensitization, respectively, of the group for sole administration of Drug A, the group for sole administration of Drug B and the group for combined administration. The edema volume for both hindlimbs (mL) is expressed in the average value ± standard error of 5 to 6 rats in each group. Furthermore, the decreasing rate is indicated as (average value of edema volume for both hindlimbs for the control group - the average value of edema volume for hindlimbs for each group)/(average value of edema volume for both hindlimbs for the control group) x 100, while the relative index is indicated as (average value of edema volume for both hindlimbs for each group)/(average value of edema volume for both hindlimbs for the control group). Also, Fig. 5 presents a diagram obtained by superposing this data with the graph of changes in the dose-response of leflunomide (0.5, 1.5, 2.5, 5 mg/kg) over time.

In conclusion, the group for sole administration of Drug A did not show a potent edema suppressive effect, and the group for sole administration of Drug B was observed to have a decrease in the edema suppressive effect in the stage after 18 days from the initial sensitization.
Meanwhile, the group for combined administration of both drugs was observed to have an extension of the duration of edema suppression which was comparable to that of 3 mg/kg of leflunomide. Also, in the group for combined administration of both drugs, the respective relative indices after 18, 21, 26 and 31 days from the initial sensitization were smaller than the product of the relative indices of the respective groups for sole administration. Thus, a clear synergistic effect due to the combined use was demonstrated.

**[Table 1] 18 days after initial sensitization**

| Test drug | Edema volume for both hindlimbs (mL) | Decreasing rate (%) | Relative index |
|---|---|---|---|
| Control group | 2.01±0.25 | | |
| Group for sole administration of Drug A (3 mg/kg) | 1.79±0.17 | 11 | 0.89 |
| Group for sole administration of Drug B (1.5 mg/kg) | 0.57±0.32 | 72 | 0.28 |
| Group for combined | 0.07±0.03 | 97 | 0.03 |
| administration of Drug A and Drug B | | | |

| | | | |
|---|---|---|---|
| Product of relative indices of the groups for sole administration: 0.89 × 0.28 = 0.25 The edema volume for both hindlimbs represents the average value ± standard error of 5 to 6 rats in each group. | | | |

**[Table 2] 21 days after initial sensitization**

| Test drug | Edema volume for both hindlimbs (mL) | Decreasing rate (%) | Relative index |
|---|---|---|---|
| Control group | 1.71±0.19 | | |
| Group for sole administration of Drug A (3 mg/kg) | 1.54±0.17 | 10 | 0.90 |
| Group for sole administration of Drug B (1.5 mg/kg) | 0.58±0.31 | 66 | 0.34 |
| Group for combined administration of Drug A and Drug B | -0.06±0.05 | 104 | -0.04 |

| | | | |
|---|---|---|---|
| Product of relative indices of the groups for sole administration: 0.90 × 0.34 = 0.30 The edema volume for both hindlimbs represents the average value ± standard error of 5 to 6 rats in each group. | | | |

**[Table 3] 26 days after initial sensitization**

| Test drug | Edema volume for both hindlimbs (mL) | Decreasing rate (%) | Relative index |
|---|---|---|---|
| Control group | 1.59±0.19 | | |
| Group for sole administration of Drug A (3 mg/kg) | 1.58±0.27 | 1 | 0.99 |
| Group for sole administration of Drug B (1.5 mg/kg) | 0.78±0.28 | 51 | 0.49 |
| Group for combined administration of Drug A and Drug B | 0.40±0.17 | 75 | 0.25 |

| | | | |
|---|---|---|---|
| Product of relative indices of the groups for sole administration: 0.99 × 0.49 = 0.49 The edema volume for both hindlimbs represents the average value ± standard error of 5 to 6 rats in each group. | | | |

**[Table 4] 31 days after initial sensitization**

| Test drug | Edema volume for bothhindlimbs (mL) | Decreasing rate (%) | Relative index |
|---|---|---|---|
| Control group | 1.41±0.14 | | |
| Group for sole administration of Drug A (3 mg/kg) | 1.44±0.26 | -2 | 1.02 |
| Group for sole administration of Drug B (1.5 mg/kg) | 0.81±0.26 | 43 | 0.57 |
| Group for combined administration of Drug A and Drug B | 0.52±0.25 | 63 | 0.37 |

| | | | |
|---|---|---|---|
| Product of relative indices of the groups for sole administration: 1.02 × 0.57 = 0.58 The edema volume for both hindlimbs represents the average value ± standard error of 5 to 6 rats in each group. | | | |

## Claims

1. A prophylactic and/or therapeutic agent for rheumatoid arthritis, comprising 2-benzyl-5-(4-chlorophenyl)-6-[4-(methylthio)phenyl]-2H-pyr idazin-3-one and leflunomide in combination.

2. The prophylactic and/or therapeutic agent for rheumatoid arthritis according to claim 1, wherein the rheumatoid arthritis is accompanied by articular inflammation.

3. The prophylactic and/or therapeutic agent for rheumatoid arthritis according to claim 1 or 2, which is for oral administration.

4. Use of 2-benzyl-5-(4-chlorophenyl)-6-[4-(methylthio)phenyl]-2H-pyr idazin-3-one and leflunomide for manufacturing a prophylactic and/or therapeutic agent for rheumatoid arthritis.

5. The use according to claim 4, wherein the rheumatoid arthritis is accompanied by articular inflammation.

6. The use according to claim 4 or 5, wherein the prophylactic and/or therapeutic agent for rheumatoid arthritis is for oral administration.

7. A prophylactic and/or therapeutic method for rheumatoid arthritis, comprising administering 2-benzyl-5-(4-chlorophenyl)-6-[4-(methylthio)phenyl]-2H-pyr idazin-3-one and leflunomide.

8. The prophylactic and/or therapeutic method for rheumatoid arthritis according to claim 7, wherein the rheumatoid arthritis is accompanied by articular inflammation.

9. The prophylactic and/or therapeutic method for rheumatoid arthritis according to claim 7 or 8, wherein the means of administration is oral administration.
